# EUROPEAN PATENT APPLICATION

(11) **EP 1 214 931 A1**
(43) Date of publication of application: **19.06.2002**
(21) Application number: 00127556.9
(22) Date of filing: 15.12.2000
(51) Int. Cl.: A61K 7/48, A61K 31/00

(54) **Water-free skin care formulations comprising micronized urea and method of manufacturing the same**

(71) Applicant: Schering Aktiengesellschaft, 13353 Berlin (DE)
(72) Inventor: Franke, Patrick, Dr., 14167 Berlin (DE); Hoffmann, Karin, Dr., 13156 Berlin (DE)
(74) Representative: Dörries, Hans Ulrich, Dr.

(57) **Abstract**

The invention provides novel water-free medical skin care formulations comprising micronized urea dispersed in a fatty phase. The fatty phase consists essentially of hydrocarbons and is essentially free from ingredients with known allergenic potential such as perfumes or perfume substitutes (fragrances), preservatives, colorings, plant extracts, PEGs, cetylstearyl alcohol, lanolin alcohols, lower alcohols, and proteins, thus being particularly suitable for patients with highly sensitive skin. The formulations of the invention have favorable sensorial properties and are stable for an extended period of time. The formulations are suitable for medical skin care, such as for the treatment of severely affected dry, sensitive skin and for the therapy or therapy support of ichthyosis, psoriasis, and eczema. The invention also provides methods of manufacturing such water-free skin care formulations.

## Description

### Field of the Invention

This invention relates to water-free skin care formulations comprising micronized urea. The formulations of the invention are particularly suitable for medical applications such as for treating extremely dry, sensitive skin and for therapy of various skin conditions such as ichthyosis, eczema and psoriasis. The current invention furthermore relates to a method for producing such water-free skin care formulations comprising micronized urea.

### Background of the Invention

Human skin acts as a barrier and protective layer against chemical, physical and mechanical insults. The primary function of the skin's outer layer―the horny layer―is to prevent loss of water and vital substances into the environment while keeping noxious exogenous materials from penetrating into living tissue. The person skilled in the art refers to the outer horny layer as the *stratum corneum* (SC). Other functions of the SC include regulation of homeostatic mechanisms to assure steady-state conditions in the face of a changing environment, mechanisms of repair after disruption of the barrier by disease or exogenous insults and the presence of anti-bacterial substances to protect against infection. In order to fulfill these functions, the SC needs to be hydrated and flexible.

Complicated mechanisms in the skin maintain the essential hydration state of the SC. However, many factors work to compromise the SC barrier and increase the rate of water loss. Exposure to harsh conditions, including cold, dry winter weather, frequent washing with soap and hot water, exposure to surfactants or irritating chemicals or solvents may cause skin dryness. Apart from external factors provoking dry skin conditions, several diseases are known that are characterized by an abnormal condition or behavior of the SC. Examples of these diseases are atopic dermatitis, psoriasis and ichthyosis.

The SC sheds clusters of cells known as corneocytes from the surface of the skin while continuously replenishing them from underlying layers. This process is usually imperceptible due to the small size of the clusters, but in dry skin, the larger aggregates of these cells are shed as scales or flakes. Dry skin is characterized by a rough, dry feeling and flaky appearance. It may itch and become red and irritated, and persons suffering from very dry skin (xerosis) may scratch affected areas repeatedly. If such conditions are untreated, severely dry skin may crack and bleed. Accumulation of thick brittle scales may occur such as in psoriasis and various forms of ichthyosis.

Skin moisturizers are used to help prevent skin from becoming dry or to return dry skin to its normal condition. Several compounds are known to act as skin moisturizers or humectants. Among these are glycerol (glycerin), urea, petrolatum (petroleum jelly), polyethylene glycols (PEGs), lanolins (wool wax), lactic acid (lactate), and ceramides. In skin care products, these ingredients are generally formulated together with a large variety of other ingredients used for maximizing efficacy, compliance, stability and shelf life. Typically, skin care products are comprised of about 20-50 different ingredients, since it is the experience of the person skilled in the art that favorable results may not be achieved by formulations with fewer ingredients.

However, a common problem associated with skin care products is that they cause adverse effects in consumers or patients. Such unwanted effects include irritation (subjective and objective), allergic contact dermatitis, photocontact dermatitis, and immediate contact reactions ("*Dry Skin and Moisturizers: Chemistry & Function",* p. 403, Eds. Loden & Maibach, 2000). The most frequently observed side effect is allergic contact dermatitis. This reaction is usually caused by fragrances and preservatives present in these products. Another origin of allergic reactions are moisturizers such as lanolin and its derivatives, emulsifiers such as cocamidopropyl betaine, and certain emollients.

Although many of the ingredients typically used in skin care formulations have a known irritation or allergenic sensitization potential and the rate of allergenic reactions in consumers or patients has increased dramatically over the last few years, these ingredients are still used in the majority of products in the skin care market of today. Several skin care formulations claim to be low in certain allergens and/or irritants. U.S. Patent No. 5,863,546 discloses cosmetic compositions that exclude certain allergens and irritants.

Petrolatum belongs to the group of dermatologic ingredients known to be non-comedogenic and non-irritant. It has been used as a skin care product since its discovery by R. Chesebrough in 1872 and is even today widely used as a classic skin moisturizer in topical formulations. It blocks the evaporation of water from the skin (trans-epidermal water loss, TEWL), therefore keeping the SC well hydrated. Ghadially and co-workers described the effect of petrolatum on damaged skin demonstrating that it permeates throughout the SC intersticies, allowing normal barrier recovery despite its occlusive properties (Ghadially et al., 1992, *J. Am. Acad. Dermatol.*, 26, 387-96).

U.S. Patent No. 5,552,147 discloses cosmetic compositions containing alpha hydroxy carboxylic acids dispersed in petroleum jelly with the aid of a phosphatide.

Urea has been known for a long time to be effective as a moisturizing compound in the treatment of dry and hyperkeratotic skin and is typically used in concentrations of up to 20 % w/w. It has been found that urea-containing formulations can reduce epidermal DNA synthesis, induce epidermal differentiation, and, particularly after long-term treatment, decrease TEWL (Pigatto et al., 1996, *J. Dermatol. Treatm.*, 7, 171-75; and Hagemann & Proksch, 1996, *Acta Derm. Venereol.,* 76, 353-56). In U.S Patent No. 5,561,166, Sattler et al., a composition containing urea and salts of lactic acid for the treatment of dry skin is disclosed. Urea is known as a normal physiological metabolite and is generally regarded as nontoxic. Despite its wide use in dermatological preparations, no report on sensitization can be found in the literature.

Urea is hygroscopic and freely soluble in water. However, in an aqueous solution, it slowly hydrolyzes to ammonia and carbon dioxide.

Therefore, aqueous urea-containing moisturizing formulations, such as water-in-oil and oil-in-water emulsions, are difficult to stabilize or need to be stored in a refrigerator to prevent or reduce the decomposition of the urea.

It is known to those skilled in the art that the decomposition of urea in topical formulations can be avoided by preparing water-free formulations wherein the urea is dispersed in solid form in a hydrophobic phase. Such a water-free urea-containing formulation provides an advantage over aqueous formulation in that the solid urea is highly effective and at the same time protected from decomposition.

However, the solid urea dispersed in the hydrophobic phase often tends to recrystallize or to form larger aggregates after partial solvatation. Depending on the composition of the ointment base, the manufacturing process, and the age of the formulation, it is often observed that the urea particles in such a water-free formulation form larger crystals or aggregates which are clearly perceptible on the skin thus providing an unwanted "sand paper" effect to the skin of the patient. The latter problem seems to be responsible for the fact that only very few urea-containing water-free skin care formulations are currently marketed, e.g. Basodexan® (Kurt Herrmann GmbH, Germany), despite the known efficacy of this type of formulation.

The prior art has failed to provide a water-free urea-containing skin care formulation that strictly avoids irritant and allergenic ingredients but retains its favorable properties, in particular in the field of medical skin care. This may be attributed to the fact that avoiding potentially irritant and allergenic ingredients undermines other desired properties such as efficacy, stability, and patient/consumer compliance.

Accordingly, there is a need for an effective water-free urea-containing formulation that is non-irritant / hypoallergenic and that avoids recrystallization of the solid urea or the formation of urea aggregates.

### Object and Summary of the Invention

It is an object of the present invention to solve the problems associated with available and known water-free urea-containing formulations by providing novel water-free skin care formulations comprising urea wherein the micronized and homogenously dispersed urea does not recrystallize or form aggregates even after extended storage thus being non-perceptible upon topical application.

Moreover, the formulations of the invention are hypoallergenic and non-irritant and effectively moisturize dry, sensitive skin. Finally, the formulations of the current invention are physicochemically / microbiologically stable, odor-free and sensorially pleasing to the patient.

The new formulations are compliance-friendly and superior to previously known urea-containing water-free formulations claiming to be hypoallergenic and non-irritant and are suitable for medical skin care, such as for the treatment of dry, sensitive skin and for the therapy or therapy support of ichthyosis, psoriasis, and eczema. They are particularly suitable for application in severely affected, exposed areas of the body, such as hands, elbows, and knees.

In one aspect, the present invention provides water-free formulations comprising micronized urea dispersed in a fatty phase. The fatty phase consists essentially of hydrocarbons and is essentially free from ingredients such as perfumes or perfume substitutes (fragrances), preservatives, colorings, plant extracts, PEGs, cetylstearyl alcohol, lanolin alcohols, lower alcohols, proteins and other substances with known allergenic potential, thus being particularly suitable for patients with highly sensitive skin.

In another aspect of the invention, the skin care formulations of the invention may further comprise one or more pharmaceutically active compounds, vitamins or vitamin analogs, and/or sunscreens.

The invention furthermore provides a method for the prophylaxis and treatment of dry, sensitive skin and/or for therapy or therapy support of ichthyosis, eczematous and psoriatic skin by topically administering a therapeutically effective amount of the dermatological skin care formulations of the present invention.

In yet another aspect of the invention, a new method of manufacturing the skin care formulations of the invention is provided. The method of manufacturing according to the present invention allows a homogenous dispersion of the micronized urea without producing aggregates or larger crystals of urea during or after the manufacturing process. Furthermore, the method disclosed herein below prevents unwanted recrystallization of urea in the formulation even after extended storage.

The formulations obtainable by using the manufacturing process of the present invention are superior since they are stabilized by the homogenous solid urea matrix thus preventing an unwanted "bleeding" effect of the hydrophobic phase.

### Detailed Description of the Invention

The invention provides hypoallergenic and non-irritant water-free skin care formulations comprising micronized urea that have superior properties. The formulations of the invention, exemplary embodiments of which are described in more detail below, are particularly suitable for medical skin care and for the treatment of severely dry, sensitive skin. Despite being essentially free from such ingredients as fragrances, preservatives, colorings, plant extracts, PEGs, cetylstearyl alcohol, lanolin alcohol, lower alcohols and proteins, the formulations of the invention unexpectedly retain many of the desirable properties.

Although many of the ingredients commonly used in skin care formulations are not present, the skin care formulations of the invention are highly effective with maximal skin tolerance and are suitable for application in climate zone IV.

Despite being particularly suitable for medical use in the treatment of severely affected dry skin, the skin care formulations of the invention have favorable sensorial properties. The micronized solid urea dispersed in the hydrophobic phase of the formulations is not perceptible and provides a soft feeling to the skin.

The formulations of the invention furthermore exhibit excellent physical/microbiological stability (see Example 3) and fulfill the requirements of the cosmetic directives of the European Union and the United States. The formulations are physicochemically stable and meet the United States Pharmacopoeia (USP) requirements in terms of microbiological stability. Although free of any preservatives, a shelf life of at least 2 years can be expected for the formulations of the invention.

In one embodiment of the invention, a water-free medical skin care formulation comprising micronized urea dispersed in a fatty phase is provided which is essentially free from fragrances, preservatives, colorings, plant extracts, PEGs, cetylstearyl alcohol, lanolin alcohol, lower alcohols, and proteins. With "essentially free" it is meant that the formulations can only contain such amounts of substances with a known allergenic potential that are known to be non-irritant and hypoallergenic. Preferably, the formulations of the invention are completely devoid of such substances.

The hydrophobic or fatty phase of the formulations consists essentially of hydrocarbons. Preferably, the fatty phase is comprised of more than 80 % (w/w), more preferably more than 85 % (w/w), and most preferably more than 90 % (w/w) of hydrocarbons.

In a preferred embodiment, the dispersed urea particles in the formulations have a homogenous particle size distribution with a particle size of less than about 100 µm, preferably less than about 70 µm.

In another preferred embodiment, the hydrophobic or fatty phase of the water-free medical skin care formulations comprises the ingredients petrolatum, paraffinum liquidum, and/or microcrystalline wax, and more preferably, consists of one or more of these ingredients.

In a particularly preferred embodiment, the hydrophobic phase of the skin care formulations may further comprise oleyl erucate.

Optionally, the water-free medical skin care formulation of the invention may further contain a conventional drying agent. It is known to those skilled in the art that a drying agent may improve the handling during the micronization process of hygroscopic substances and may lead to a higher quality in micro- and macroscopic appearance. Any suitable drying agent known in the art can be used in the practice of the invention. Maize starch is a particularly suitable and preferred drying agent that can be added to the urea prior to the micronization process.

The formulations of the invention have relatively few ingredients, preferably less than 9, most preferably 6 or 7 ingredients.

In one preferred embodiment of the present invention, the water-free skin care formulation is in the form of a semi-solid ointment which is particularly suitable for topical application to severely affected or diseased exposed areas of the body. Such a preferred formulation comprises the following ingredients in amount ranges expressed as percent by weight (% w/w):

| | | |
|---|---|---|
| a) | petrolatum | 50 - 65 % |
| b) | paraffinum liquidum | 15 - 25 % |
| c) | oleyl erucate | 4 - 6 % |
| d) | microcrystalline wax | 9 - 11 % |
| e) | micronized urea | 0.5 - 10 % |
| f) | maize starch | 0 - 1 % |

Particularly preferred is a water-free skin care formulation in the form of a semi-solid ointment, wherein the formulation comprises the following ingredients expressed as percent by weight (% w/w):

| | | |
|---|---|---|
| a) | petrolatum | 59.5 % |
| b) | paraffinum liquidum | 20 % |
| c) | oleyl erucate | 5 % |
| d) | microcrystalline wax | 10% |
| e) | micronized urea | 5 % |
| f) | maize starch | 0.5 % |

This formulation will hereinafter be referred to as "Formulation A"

In general, the ingredients used for the medical skin care formulations of the invention should be of high quality and purity.

Additionally, the formulations of the invention may further comprise one or more of the ingredients selected from a sunscreen, a vitamin or vitamin analog, such as Vitamin E, Vitamin K, Calcipotriol, Tacalcitol, or a pharmaceutically active compound, such as corticoids, antimycotics, immunosuppressants, salicylic acid, alpha- or beta-hydroxy acids, or linolenic acid. The group of corticoids may include but is not limited to hydrocortisone and derivatives thereof, such as methylprednisolone aceponate, diflucortolone valerate, fluocortolone pivalate / hexanoate / monohydrate, fluocortinbutyl, betamethasone and derivatives thereof, clobetasole, triamcinolone acetonide, dexamethasone, prednisolone, and clocortolone pivalate. The group of antimycotics may include but is not limited to ketoconazole, isoconazole, miconazole, and clotrimazole; and the group of immunosuppressants may include but is not limited to ascomycin and derivatives thereof, cyclosporine A, azathioprine, methotrexate, cyclophosphamide, and the like.

Due to their favorable properties, the novel skin care formulations of the present invention are particularly suitable for use as emollients, in the therapy or therapy support of ichthyosis, eczematous skin and psoriatic skin, as well as in therapeutic daily skin care treatment of extremely dry, sensitive skin.

The invention furthermore provides a robust and easily applicable method of manufacturing the skin care formulations of the invention. The manufacturing method comprises the steps of:
(a) micronizing the urea, optionally in the presence of a drying agent such as maize starch.
(b) preparing a hydrophobic or fatty phase, by melting the ingredients of the fatty phase, such as petrolatum, paraffinum liquidum, microcrystalline wax, and oleyl erucate.
(c) homogenizing and stirring the micronized urea of step (a) and the heated fatty phase of step (b) at melting temperature while applying a vacuum and cooling the formulation down to below 35°C after homogenization with continued stirring.

In step (a), micronization of urea is conveniently achieved by using known milling devices such as a jet mill. After milling, the urea particles preferably have a particle size of less than 100 µm, more preferably less than 70 µm.

In step (b), the selected ingredients are heated in a suitable melting vessel with stirring until they are completely melted. The ingredients chosen for the fatty phase will depend on the fatty phase that is desired, as described above. The fatty phase is preferably heated to about 75 - 90°C during the melting process, although the skilled person will realize that the necessary melting temperature depends on its exact composition. Prior to the combination of the hydrophobic phase of step (b) and the micronized urea of step (a), the hydrophobic phase can be filtered through filter cartridges with 0.1 mm filter units for particulate filtration.

In a preferred method, the homogenization of step (c) is accomplished by using an ointment mixer equipped with an Ultra-Turrax™-type homogenizer, a dissolver disc, and a variable stirrer.

In a particularly preferred manufacturing method, the homogenization step (c) is done in the above ointment mixer and comprises the steps of:
(i) homogenizing the micronized urea and the fatty phase with a gap diameter suitable for homogenization at high speed under a vacuum of about -0,2 to -0.4 bar and at a temperature of 83 - 87°C while stirring;
(ii) reducing the gap diameter to about half of the starting diameter and continuing homogenization with otherwise unchanged parameters concerning temperature, homogenizing/stirring speed and temperature.

In general, a suitable starting gap diameter for dispersing solid urea in a melted fatty phase may range from 0.2 to 0.6 mm, more preferably from 0.3 to 0.5 mm. However, the skilled person will appreciate that the gap diameter of the Ultra-Turrax™ homogenizer may vary depending on the particular model used for homogenization and the batch size of the manufacturing process.

After the cool-down process, the formulations of the present invention can be discharged into appropriate containers.

The invention is further illustrated in the Examples. The Examples are not intended to limit the scope of the invention.

### Example 1

### Preparation of a water-free skin care formulation comprising micronized urea

A formulation was prepared in a batch size of 100 kg using an ointment mixer with a 300 L stainless steel pan and an Ultra-Turrax homogenizer (e.g., Fryma® system from Fryma AG, Switzerland), a stainless steel melting pan equipped with an infinitely variable stirrer, stainless steel ointment storage containers, and stainless steel sintered filter cartridges, 0.1 mm for particulate filtration.

5.0 kg urea and 0.50 kg maize starch were sieved and mixed together. The mixture was continuously micronized using a spiral type jet-mill (200 mm) until the particle size was < 70 µm.

The fatty phase was prepared by charging the melting vessel with 59.5 kg petrolatum, 20.0 kg paraffinum liquidum, 5.00 kg oleyl erucate, 10.00 kg microcrystalline wax and melted completely at 85 ± 2 °C while stirring until no unmelted material remained visible. Finally, the fatty phase was filtered through stainless steel filter cartridges with 0.1 mm filter units for particulate filtration prior to the addition of the urea / maize starch mixture.

The fatty phase was then transferred to the ointment mixer at 85 ± 2 °C and, after turning on the dissolver disc at high speed while stirring at 40 min⁻¹, the micronized urea / maize starch mixture was subsequently added to the mixer. The gap diameter was then adjusted to about 0.4 mm and homogenization was started by turning on the Ultra-Turrax homogenizer at high speed with a running dissolver disc (high speed) and stirrer (∼40 min⁻¹) at 85 ± 2°C while applying a vacuum of about -0.3 bar. After 10 minutes, the gap diameter was adjusted to about 0.2 mm and the homogenization was continued for another 10 minutes under the same conditions as described above.

The ointment was checked for homogeneity by microscopy and the homogenizer and the dissolver disc were turned off after 20 minutes of homogenization. The ointment mixture was finally cooled down to 30°C± 2°C (while applying a vacuum of -0,3 bar) with continuous stirring (∼40 min⁻¹) and discharged into bulk containers.

### Example 2

### Alternative preparation of a water-free skin care formulation

A formulation was prepared in a batch size of 50 kg using an ointment mixer and homogenizer (available under the tradename Becomix® RW 60 from Berents, Germany), equipped with a 60 L stainless steel pan, an infinitely variable anchor-type stirrer, homogenizer and process control system, a pH-meter, a stainless steel melting pan equipped with an infinitely variable stirrer, stainless steel ointment storage containers, and stainless steel sintered filter cartridges, 0.1 mm for particulate filtration.

In a first step, 2.5 kg urea and 0.25 kg maize starch were micronized as described in Example 1.

The fatty phase was prepared by charging the melting vessel with 29.75 kg petrolatum, 10.0 kg paraffinum liquidum, 2.5 kg oleyl erucate, 5.0 kg microcrystalline wax and melted completely at 80 ± 2 °C while stirring until no unmelted material remained visible. Finally, the fatty phase was filtered through stainless steel filter cartridges with 0.1 mm filter units for particulate filtration prior to the addition of the urea / maize starch mixture.

The ointment was then prepared by transferring the micronized urea / maize starch mixture to the ointment mixer/homogenizer while homogenizing (1000 min⁻¹, inline, left direction) at 80 ± 2 °C. After charging the ointment mixer/homogenizer with the urea mixture, the resulting mixture was stirred (40 min⁻¹) under a vacuum of less than about -0,7 bar. The homogenization process was started by homogenizing for 5 min at 2500 min⁻¹ (inline, left direction), followed by homogenizing for 5 min at 4500 min⁻¹ (inline, right direction) while stirring (40 min⁻¹, left), then for 5 min at 4500 min⁻¹ (inline, left direction) while stirring (40 min⁻¹, left direction), then for 5 min at 4500 min⁻¹ (cycle, left direction) while stirring (40 min⁻¹, right direction), and finally for 5 min at 4500 min⁻¹ (cycle, right direction) while stirring (40 min⁻¹, right direction).

The ointment was checked for homogeneity by microscopy to ensure uniform appearance. The resultant ointment was then cooled to 30°± 2°C while it was stirred at 40 min⁻¹. During the cool down process a vacuum of between -0.6 and -0.9 bar was applied. After the cool down process, the formulation was discharged into bulk containers.

### Example 3

### Physicochemical / microbiological stability

The formulations as prepared in Examples 1 and 2 were subjected to the following stability tests:
a) Storage at 4°C, 25°C, 30°C (70% rel. humidity) over 12 months and 40°C (75% rel. humidity) over 6 months.
b) Microbiological challenge test according to USP (United States Pharmacopoeia) requirements.

The formulations were packaged in PE containers (COEX) in 100 kg batch sizes and tested for parameters such as microscopic and macroscopic picture, content of urea, and compatibility with the container material.

The test results showed that the formulations as prepared according to Example 1 and 2 are physicochemically stable under all tested conditions and meet the microbiological stability requirements of the USP.

### Example 4

### Sensitization Risk and Irritation Potential

A 21-Day cutaneous cumulative irritancy test following repeated applications on human healthy skin was carried out with the medical skin care formulation as prepared in Example 1. The study was designed as a single center, randomized, double-blind (investigator-masked) study with intraindividual comparison of treatments to evaluate the cutaneous tolerance of repeated applications of the medical skin care formulations applied under occlusion during 21 days and started with 25 healthy volunteers of either sex between 18 and 65 years old meeting specific inclusion / exclusion criteria.

The formulation of Example 1 was compared intraindividually with two reference products, one being a commercially available skin base cream and the other being white soft paraffin.

The volunteers were subjected to an occlusive application of 50 µl of each test formulation on designated test fields on their mid back for 21 consecutive days. The occlusive dressings were removed after 23 hours, and the irritation was evaluated one hour later using a 6-point-score (irritation score):

| Visual 6-point-score | |
|---|---|
| 0 | No erythema |
| 0.5 | Equivocal erythema |
| 1 | Slight erythema, with and without edema |
| 2 | Moderate erythema, edema with or without papules |
| 3 | Severe erythema, edema with or without papules |
| 4 | Severe erythema, edema with vesicles or blisters |

A Cumulative Irritancy Index (C.I.I.) was calculated for each test preparation for each subject as follows:
- C.I.I. = Sum of irritation scores in one individual/ number of readings

An 'adjusted C.I.I.' (C.I.I.*) was calculated by subtracting scores caused by sensitization reactions.

Furthermore, a Mean Cumulative Irritation Index (M.C.I.I.) was calculated for each product by averaging individual C.I.I.s across subjects as follows:
- M.C.I.I. = Sum of irritation scores in all individuals/ number of readings

An 'adjusted M.C.I.I.' (M.C.I.I.*) was calculated by subtracting scores caused by sensitization reactions.

### Results:

No serious adverse event related to the test formulations occurred during the course of the study.

### Tolerability / Irritation potential:

No increase in the individual scores, C.I.I., and C.I.I.* could be observed during the course of the study for the medical skin care formulation of Example 1. Regarding the control formulation and paraffin, no remarkable differences or tendencies in mean scores during the course of the study (except for the first days) could be observed whereas the formulation of Example 1 tended to lower values of C.I.I. and C.I.I.*.

With regard to the M.C.I.I.* and the classification of the irritation potential the formulation of Example 1 was classified as non-irritant.

### Sensitization:

No case of sensitization was observed for the medical skin care formulation of Example 1 in any of the volunteers during the course of the study. The formulation of Example 1 thus possesses an excellent sensitization profile.

## Claims

1. A water-free medical skin care formulation comprising micronized urea dispersed in a fatty phase which is essentially free from fragrances, preservatives, colorings, plant extracts, PEGs, cetylstearyl alcohol, lanolin alcohol, lower alcohols and proteins, wherein the fatty phase consists essentially of hydrocarbons.

2. The water-free medical skin care formulation according to claim 1, wherein the dispersed urea particles have a homogenous particle size distribution with a particle size of less than about 100 µm.

3. The water-free medical skin care formulation according to claims 1 or 2, wherein the fatty phase comprises petrolatum, paraffinum liquidum, and/or microcrystalline wax.

4. The water-free medical skin care formulation according to any of claims 1 to 3, wherein the formulation further comprises oleyl erucate.

5. The water-free medical skin care formulation according to any of claims 1 to 4, wherein the formulation further comprises a drying agent, preferably maize starch.

6. The water-free medical skin care formulation according to any of claims 1 to 5, the formulation having a total number of ingredients that is less than 9.

7. The water-free medical skin care formulation according to any of claims 1 to 6, wherein the formulation comprises the following ingredients expressed as a percent by weight (% w/w) range:
| | | |
|---|---|---|
| a) | petrolatum | 50 - 65 % |
| b) | paraffinum liquidum | 15 - 25 % |
| c) | microcrystalline wax | 9 - 11 % |
| d) | oleyl erucate | 4 - 6 % |
| e) | micronized urea | 0.5 - 10 % |
| f) | maize starch | 0 - 1 % |

8. The water-free medical skin care formulation according to claim 7, wherein the formulation comprises the following ingredients expressed as percent by weight (% w/w):
| | | |
|---|---|---|
| a) | petrolatum | 59.5 % |
| b) | paraffinum liquidum | 20 % |
| c) | microcrystalline wax | 10 % |
| d) | oleyl erucate | 5 % |
| e) | micronized urea | 5 % |
| f) | maize starch | 0.5 % |

9. The skin care formulation according to any of claims 1 - 8, wherein the formulation further comprises one or more pharmaceutically active compounds, vitamins or vitamin analogs, and/or sunscreens.

10. The skin care formulation according to claim 9, wherein the pharmaceutically active compound is a corticoid, an antimycotic, an immunosuppressant, salicylic acid, an alpha- or beta-hydroxy acid, and/or linolenic acid; wherein the vitamin is Vitamin E or Vitamin K; and wherein the vitamin analog is Calcipotriol or Tacalcitol.

11. The skin care formulation according to any of claims 1 - 10 for use in the prophylaxis and treatment of dry, sensitive skin and/or in the therapy or therapy support of ichthyosis, eczematous skin and psoriatic skin.

12. A method of manufacturing a water-free skin care formulation comprising the steps of:
a) micronization of urea, optionally in the presence of a drying agent;
b) preparing a fatty phase by melting the ingredients of the fatty phase as defined in any of claims 1-8;
c) homogenizing and stirring the micronized urea of step (a) and the heated fatty phase of step (b) at melting temperature while applying a vacuum and cooling the formulation down to below 35°C after homogenization with continued stirring.

13. The method according to claim 12, wherein the homogenization step (c) is performed in an ointment mixer equipped with an Ultra-Turrax-type homogenizer, a dissolver disc and a variable stirrer.

14. The method according to claim 13, wherein the homogenization step (c) comprises the steps of:
(i) homogenizing the micronized urea and the fatty phase with a gap diameter suitable for homogenization at high speed under a vacuum of about -0,2 to - 0.4 bar and at a temperature of 83 - 87°C while stirring;
(ii) reducing the gap diameter to about half of the starting diameter and continuing homogenization with otherwise unchanged parameters regarding temperature, homogenizing/stirring speed and temperature.

15. A water-free medical skin care formulation obtainable by any of the methods of claims 12 to 14.
